# EUROPEAN PATENT APPLICATION

(11) **EP 4 151 734 A1**
(43) Date of publication of application: **22.03.2023**
(21) Application number: 20935947.0
(22) Date of filing: 11.05.2020
(51) Int. Cl.: C12N 15/85, C07K 16/42

(54) **HUMAN GENOME-DERIVED POLYNUCLEOTIDE AND METHOD FOR PRODUCING POLYPEPTIDE OF INTEREST USING SAME**

(71) Applicant: Samsung Bioepis Co., Ltd., Yeonsu-gu Incheon 21987 (KR)
(72) Inventor: RYU, Sung Jin, Incheon 21987 (KR); PARK, Sangjoon, Incheon 21987 (KR); KIM, Sunkyu, Incheon 21987 (KR); LIN, Powei, Incheon 21987 (KR); KANG, Eunji, Incheon 21987 (KR); KIM, Young Soo, Incheon 21987 (KR); KIM, Hyung-Chan, Incheon 21987 (KR); LEE, Ju Hee, Incheon 21987 (KR); LEE, Chanmoo, Incheon 21987 (KR); JEONG, Yun Ju, Incheon 21987 (KR)
(74) Representative: Grünecker Patent- und Rechtsanwälte PartG mbB
(86) International application number: PCT/KR2020/006193
(87) International publication number: WO 2021/230391

(57) **Abstract**

Provided are a human genome-derived polynucleotide having a chromatic regulator factor function, a recombinant vector and a recombinant cell, each comprising same, and a method for producing a polypeptide of interest using same.

## Description

### Technical Field

Provided are a human genome-derived polynucleotide, a recombinant vector carrying same, a recombinant cell anchoring same thereat, and a method for producing a polypeptide of interest using same.

### Background Art

For mass production of therapeutic proteins, advantage is taken of genetic recombination technology and a variety of cells, as host cells, ranging from microorganisms such as E. coli to mammalian cells. Among therapeutic proteins, glycoproteins are often produced to normal activity only when expressed from mammalian cells due to processes such as eukaryotic glycosylation.

On the whole, therapeutic protein-producing cells are obtained as a stable expression system in which a foreign gene is introduced into a host cell and incorporated into the chromosome thereof to make continual expression even over passages. In this regard, the incorporation into a specific position of the chromosome does not occur in every trial, as a general rule. Chromatin is a constituent of mammalian genomic DNA. The organization of the chromatic may be classified as either euchromatin or heterochromatin, depending on its level of compaction. Euchromatin is the lightly packed form of chromatin, allowing for the active expression of proteins whereas heterochromatin refers to a more densely packed form in which protein expression is thus less apt to take place. The random insertion of an expression vector into the chromosome leaves the expression level to luck depending on which of the euchromatin and the heterochromatin the vector is inserted into. When an expression vector is inserted into a heterochromatin, the expression level decreases over time, resulting in a silencing phenomenon.

Mass production in animal cells, unlike microbial systems, requires a technology of transforming host cells for stable insertion of a foreign gene into the chromosome of the host cells. However, such transformed cells are very low, for the most part, in expression frequency. In addition, even when the transformed cells are selected, the insertion position of the gene differs from one cell to another and thus the expression pattern varies depending on the insertion position, which is known as "position effect".

The expression pattern of a foreign gene is dependent on whether the chromatin around the insertion position of the gene is wrapped loosely to allow the expression of the gene to be active (e.g., a foreign gene is inserted into an euchromatin region) or tightly to make the expression inactive (e.g., a foreign gene is inserted into a heterochromatin region). In order to acquire a great deal of protein expression by surmounting the silencing phenomenon that the expression of a foreign gene is inactivated upon its insertion into a heterochromatin there is a need for discovery of a factor that makes the chromatin structure more open to turn the inactive chromatin into an active state, thereby allowing a foreign gene to be expressed irrespective of the insertion position and for a gene expression technology using same.

### Disclosure

### Technical Problem

The present disclosure aims to provide a human genomic DNA-derived polynucleotide serving as a chromatin control element (CCE) wherein the chromatin control element functions to make the chromatin structure loose to activate the inactive chromatin, whereby a foreign gene can be expressed at an elevated level irrespectively of the insertion position of the foreign gene, with the consequent increased production of the polypeptide encoded by the foreign gene introduced into a host cell.

In an aspect of the present disclosure, the polynucleotide may comprise at least one selected from the group consisting of:
a) a polynucleotide comprising 100 or more consecutive nucleotides within a nucleotide sequence selected from among SEQ ID NOS: 1 to 16;
b) a polynucleotide, comprising 200 to 5,000 consecutive nucleotides inclusive of the polypeptide a), on human chromosome X;
c) a polynucleotide comprising a nucleotide sequence complementary to the polynucleotide a) or b); and
d) a polynucleotide comprising a nucleotide sequence having an identity of 80% or higher with the polynucleotide a), b), or c).

Another aspect provides a recombinant vector carrying the polynucleotide. The recombinant vector may further comprise typical regulatory elements of gene expression, such as a promoter, a transcriptional terminator, and so on, in addition to the polynucleotide. In this regard, the polynucleotide may be located at the 5' end of a promoter, the 3' end of a transcriptional terminator, or both of the ends. When located at both of the ends, the polypeptide may be arranged in opposite directions. The recombinant vector may further comprise a gene coding for the polypeptide of interest between a promoter and a terminator.

A further aspect provides a recombinant cell having the recombinant vector introduced into a host cell therefor. The recombinant cell may have a gene encoding a polypeptide of interest introduced between a promoter and a transcriptional terminator.

A still further aspect provides a method for producing a polypeptide of interest or for enhancing the production of a polypeptide of interest, the method comprising a step of expressing a gene coding for the polypeptide of interest in the recombinant cell.

Still another aspect provides a method for producing a polypeptide of interest or for enhancing the production of a polypeptide of interest, the method comprising a step of introducing the polynucleotide and a gene coding for the polypeptide of interest into a host cell.

Yet another aspect provides a composition for producing a polypeptide of interest or for enhancing the production of a polypeptide of interest, the composition containing at least one selected from the group consisting of the polynucleotide, a recombinant vector carrying the polynucleotide, and a recombinant cell anchoring the polynucleotide or the recombinant vector thereat.

### Technical Solution

The present disclosure provides a newly discovered polynucleotide derived from human genomic DNA and a use thereof. The polynucleotide acts as a chromatin control element (CCE) to make the chromatin structure loose when introduced into a host cell, allowing for the stable expression pattern of a foreign gene inserted into the host cell irrespective of the insertion position thereof, with the consequent improvement in the production of the polypeptide encoded by the foreign gene, compared to the absence of the polynucleotide.

The newly discovered polynucleotide may control the degree of compactness (looseness and tightness) of the surrounding chromosome structure in the host cell, thereby activating the transcription of genes in the corresponding region. The artificial introduction of the polynucleotide into a host cell can stabilize the expression of a recombinant gene in the host cell. The transcriptional activation function of the polynucleotide may be related to the structural feature there rather than the primary nucleotide sequence thereof. For example, the polynucleotide may be high in A and T content and exhibit morphological and physicochemical properties such as the molecule's own natural curvature, narrow and minor grooves, sensitivity to denaturation, etc.

Below, as used herein, the term "polynucleotide", unless specifically mentioned, is construed to encompass any polynucleotide that has an identity of 60% or higher, 65% or higher, 70% or higher, 75% or higher, 80% or higher, 85% or higher, 90% or higher, 92% or higher, 95% or higher, 97% or higher, 98% or higher, or 99% or higher with the nucleic acid (nucleotide) sequence defined by the sequence identification number of the present disclosure as long as it retains the desired function as the chromatin control element. Provided according to an aspect is a human genomic DNA-derived polypeptide that retains a function as a chromatin control element. The polynucleotide may function to control a degree of compactness (looseness and tightness) of a surrounding chromosome structure to activate the transcription of genes in a corresponding region.

In an embodiment, human-derived cells overexpressing a certain gene (reporter gene; e.g., GFP gene, etc.) were examined for the insertion position of the reporter gene, followed by searching for elements located around the insertion position and capable of affecting the overexpression to discover a polynucleotide that can function as a chromatin control element (CCE).

The polynucleotide thus attained may be a genetic entity located at a human genome, for example, human (Homo sapiens) chromosome X ((e.g., a base pair region from 43,589,420^{th} nt. to 43,592,849^{th} nt. (SEQ ID NO: 1 and/or a complementary nucleotide sequence thereto), a base pair region from 107,130^{th} to 110,129^{th} (SEQ ID NO: 2 and/or a complementary nucleotide sequence thereto), a base pair region from 135,113,641^{st} to 135,116,640^{th} (SEQ ID NO: 3 and/or a complementary nucleotide sequence thereto), a base pair region from 135,176,090^{th} to 135,179,089^{th} (SEQ ID NO: 4 and/or a complementary nucleotide sequence thereto), a base pair region from 106,848,480^{th} to 106,851,813^{th} (SEQ ID NO: 5 and/or a complementary nucleotide sequence thereto), a base pair region from 23,829,483^{rd} to 23,832,482^{nd} (SEQ ID NO: 6 and/or a complementary nucleotide sequence thereto), a base pair region from 23,849,286^{th} to 23,853,028^{th} (SEQ ID NO: 7 and/or a complementary nucleotide sequence thereto), a base pair region from 23,884,868^{th} to 23,888,180^{th} (SEQ ID NO: 8 and/or a complementary nucleotide sequence thereto), a base pair region from 71,124,431^{st} to 71,128,430^{th} (SEQ ID NO: 9 and/or a complementary nucleotide sequence thereto), a base pair region from 23,315^{th} to 27,314^{th} (SEQ ID NO: 10 and/or a complementary nucleotide sequence thereto), a base pair region from 135,175,898^{th} to 135,179,196^{th} (SEQ ID NO: 11 and/or a complementary nucleotide sequence thereto), a base pair region from 73,285,832^{nd} to 73,288,831^{st} (SEQ ID NO: 12 and/or a complementary nucleotide sequence thereto), a base pair region from 73,299,440^{th} to 73,302,469^{th} (SEQ ID NO: 13 and/or a complementary nucleotide sequence thereto), or a base pair region from 73,322,470^{th} to 73,325,469^{th} (SEQ ID NO: 14 and/or a complementary nucleotide sequence thereto)).

Therefore, the human genomic DNA-derived polynucleotide that functions as a chromatin control element may be a human chromosomal polynucleotide that is located, for example, at the chromosome X in Homo sapiens, e.g., a nucleotide sequence selected from the group consisting of SEQ ID NOS: 1 to 14 and complementary sequences thereto, or a part thereof (SEQ ID NO: 15 or 16). In detail, the human genomic DNA functioning as a chromatin control element (CCE) may be selected from the group consisting of:
i) a polypeptide derived from a human chromosomal region, e.g., chromosome X in humans (Homo sapiens), for example, a polypeptide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, i.e., 100 to 5000, 100 to 4500, 100 to 4000, 100 to 3500, 100 to 3000, 200 to 5000, 200 to 4500, 200 to 4000, 200 to 3500, 200 to 3000, 500 to 5000, 500 to 4500, 500 to 4000, 500 to 3500, 500 to 3000, 700 to 5000, 700 to 4500, 700 to 4000, 700 to 3500, 700 to 3000, 900 to 5000, 900 to 4500, 900 to 4000, 900 to 3500, 900 to 3000, 1000 to 5000, 1000 to 4500, 1000 to 4000, 1000 to 3500, 1000 to 3000, 1200 to 5000, 1200 to 4500, 1200 to 4000, 1200 to 3500, 1200 to 3000, 1400 to 5000, 1400 to 4500, 1400 to 4000, 1400 to 3500, 1400 to 3000, 1500 to 5000, 1500 to 4500, 1500 to 4000, 1500 to 3500, 1500 to 3000, 1700 to 5000, 1700 to 4500, 1700 to 4000, 1700 to 3500, 1700 to 3000, 2000 to 5000, 2000 to 4500, 2000 to 4000, 2000 to 3500, 2000 to 3000, 2200 to 5000, 2200 to 4500, 2200 to 4000, 2200 to 3500, 2200 to 3000, 2500 to 5000, 2500 to 4500, 2500 to 4000, 2500 to 3500, 2500 to 3000, 2700 to 5000, 2700 to 4500, 2700 to 4000, 2700 to 3500, 2700 to 3000, 3000 to 5000, 3000 to 4500, 3000 to 4000, or 3000 to 3500 consecutive nucleotides within a nucleotide sequence selected from the group consisting of SEQ ID NOS: 1 to 14;
ii) a polynucleotide complementary to the polynucleotide i); and
iii) a polynucleotide having an identity of 60% or higher, 65% or higher, 70% or higher, 75% or higher, 80% or higher, 85% or higher, 90% or higher, 92% or higher, 95% or higher, 97% or higher, 98% or higher, or 99% or higher with the polynucleotide i) or ii).

In an embodiment, the number of nucleotides in the polynucleotide i) may result from counting from the 5' end of any one nucleotide sequence selected from SEQ ID NOS: 1 to 14.

The polynucleotide iii) may comprise a nucleotide sequence identical by the above-mentioned range to the polynucleotide i) or ii) as a result of a partial modification (deletion, substitution, or insertion of some nucleotides) to the intact sequences of the polynucleotide i) or ii). The partial modification is allowed as long as it occurs at such positions and/or extents as not to affect the functional and structural features of the polynucleotide i) or ii). Unless stated otherwise, the polynucleotide i) and/or ii) may be construed to comprise the scope of polynucleotide iii).

In an embodiment, the polynucleotide may be at least one selected from the group consisting of:
1) a polypeptide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 100 to 3430, 200 to 3430, 500 to 3430, 700 to 3430, 900 to 3430, 1000 to 3430, 1200 to 3430, 1400 to 3430, 1500 to 3430, 1700 to 3430, 2000 to 3430, 2200 to 3430, 2500 to 3430, 2700 to 3430, 3000 to 3430, or 3430 consecutive nucleotides within the sequence of SEQ ID NO: 1;
2) a polypeptide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 1);
3) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 100 to 3730, 200 to 3730, 500 to 3730, 700 to 3730, 900 to 3730, 1000 to 3730, 1200 to 3730, 1400 to 3730, 1500 to 3730, 1700 to 3730, 2000 to 3730, 2200 to 3730, 2500 to 3730, 2700 to 3730, 3000 to 3730, or 3730 consecutive nucleotides within the sequence of SEQ ID NO: 2;
4) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 3);
5) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, or 2700 or more consecutive nucleotides, e.g., 100 to 3000, 200 to 3000, 500 to 3000, 700 to 3000, 900 to 3000, 1000 to 3000, 1200 to 3000, 1400 to 3000, 1500 to 3000, 1700 to 3000, 2000 to 3000, 2200 to 3000, 2500 to 3000, 2700 to 3000, or 3000 consecutive nucleotides within the sequence of SEQ ID NO: 3;
6) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 5);
7) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, or 2700 or more consecutive nucleotides, e.g., 100 to 3298, 200 to 3298, 500 to 3298, 700 to 3298, 900 to 3298, 1000 to 3298, 1200 to 3298, 1400 to 3298, 1500 to 3298, 1700 to 3298, 2000 to 3298, 2200 to 3298, 2500 to 3298, 2700 to 3298, or 3298 consecutive nucleotides within the sequence of SEQ ID NO: 4;
8) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 8);
9) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 100 to 3341, 200 to 3341, 500 to 3341, 700 to 3341, 900 to 3341, 1000 to 3341, 1200 to 3341, 1400 to 3341, 1500 to 3341, 1700 to 3341, 2000 to 3341, 2200 to 3341, 2500 to 3341, 2700 to 3341, 3000 to 3341, or 3341 consecutive nucleotides within the sequence of SEQ ID NO: 5;
10) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 9);
11) a polynucleotide comprising 100 or more, 200 or more, 500 or more, 700 or more, 900 or more, 1000 or more, 1200 or more, 1400 or more, 1500 or more, 1700 or more, or 2000 or more consecutive nucleotides, e.g., 100 to 2152, 200 to 2152, 500 to 2152, 700 to 2152, 900 to 2152, 1000 to 2152, 1200 to 2152, 1400 to 2152, 1500 to 2152, 1700 to 2152, 2000 to 2152, or 2152 consecutive nucleotides within the sequence of SEQ ID NO: 6;
12) a polynucleotide comprising 200 or more, 500 or more, 700 or more, 900 or more, 1000 or more, 1200 or more, 1400 or more, 1500 or more, 1700 or more, or 2000 or more consecutive nucleotides, e.g., 200 to 5000, 200 to 4500, 200 to 4000, 200 to 3500, 200 to 3000, 200 to 2500, 500 to 5000, 500 to 4500, 500 to 4000, 500 to 3500, 500 to 3000, 500 to 2500, 700 to 5000, 700 to 4500, 700 to 4000, 700 to 3500, 700 to 3000, 700 to 2500, 900 to 5000, 900 to 4500, 900 to 4000, 900 to 3500, 900 to 3000, 900 to 2500, 1000 to 5000, 1000 to 4500, 1000 to 4000, 1000 to 3500, 1200 to 5000, 1200 to 4500, 1200 to 4000, 1200 to 3500, 1200 to 3000, 1200 to 2500, 1400 to 5000, 1400 to 4500, 1400 to 4000, 1400 to 3500, 1400 to 3000, 1400 to 2500, 1500 to 5000, 1500 to 4500, 1500 to 4000, 1500 to 3500, 1500 to 3000, 1500 to 2500, 1700 to 5000, 1700 to 4500, 1700 to 4000, 1700 to 3500, 1700 to 3000, 1700 to 2500, 2000 to 5000, 2000 to 4500, 2000 to 4000, 2000 to 3500, 2000 to 3000, or 2000 to 2500 consecutive nucleotides within Homo sapiens chromosome X inclusive of the polynucleotide 11);
13) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 100 to 3743, 200 to 3743, 500 to 3743, 700 to 3743, 900 to 3743, 1000 to 3743, 1200 to 3743, 1400 to 3743, 1500 to 3743, 1700 to 3743, 2000 to 3743, 2200 to 3743, 2500 to 3743, 2700 to 3743, 3000 to 3743, or 3743 consecutive nucleotides within the sequence of SEQ ID NO: 7;;
14) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 500 to 5000, 500 to 4500, 500 to 4000, 700 to 5000, 700 to 4500, 700 to 4000, 900 to 5000, 900 to 4500, 900 to 4000, 1000 to 5000, 1000 to 4500, 1000 to 4000, 1200 to 5000, 1200 to 4500, 1200 to 4000, 1400 to 5000, 1400 to 4500, 1400 to 4000, 1500 to 5000, 1500 to 4500, 1500 to 4000, 1700 to 5000, 1700 to 4500, 1700 to 4000, 2000 to 5000, 2000 to 4500, 2000 to 4000, 2200 to 5000, 2200 to 4500, 2200 to 4000, 2500 to 5000, 2500 to 4500, 2500 to 4000, 2700 to 5000, 2700 to 4500, 2700 to 4000, 3000 to 5000, 3000 to 4500, or 3000 to 4000 consecutive nucleotides within Homo sapiens chromosome X inclusive of the polynucleotide 13);
15) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 100 to 3313, 200 to 3313, 500 to 3313, 700 to 3313, 900 to 3313, 1000 to 3313, 1200 to 3313, 1400 to 3313, 1500 to 3313, 1700 to 3313, 2000 to 3313, 2200 to 3313, 2500 to 3313, 2700 to 3313, 3000 to 3313, or 3313 consecutive nucleotides within the sequence of SEQ ID NO: 8;
16) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 15);
17) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, 3000 or more, 3200 or more, or 3500 or more consecutive nucleotides, e.g., 100 to 3524, 200 to 3524, 500 to 3524, 700 to 3524, 900 to 3524, 1000 to 3524, 1200 to 3524, 1400 to 3524, 1500 to 3524, 1700 to 3524, 2000 to 3524, 2200 to 3524, 2500 to 3524, 2700 to 3524, 3000 to 3524, 3200 to 3524, or 3500 to 3524, or 3524 consecutive nucleotides within the sequence of SEQ ID NO: 9;
18) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, 3000 or more, 3200 or more, or 3500 or more consecutive nucleotides, e.g., 200 to 5000, 200 to 4500, 200 to 4000, 500 to 5000, 500 to 4500, 500 to 4000, 700 to 5000, 700 to 4500, 700 to 4000, 900 to 5000, 900 to 4500, 900 to 4000, 1000 to 5000, 1000 to 4500, 1200 to 5000, 1200 to 4500, 1200 to 4000, 1400 to 5000, 1400 to 4500, 1400 to 4000, 1500 to 5000, 1500 to 4500, 1500 to 4000, 1700 to 5000, 1700 to 4500, 1700 to 4000, 2000 to 5000, 2000 to 4500, 2000 to 4000, 2200 to 5000, 2200 to 4500, 2200 to 4000, 2500 to 5000, 2500 to 4500, 2500 to 4000, 2700 to 5000, 2700 to 4500, 2700 to 4000, 3000 to 5000, 3000 to 4500, 3000 to 4000, 3200 to 5000, 3200 to 4500, 3200 to 4000, 3500 to 5000, 3500 to 4500, or 3300 to 4000 consecutive nucleotides within Homo sapiens chromosome X inclusive of the polynucleotide 17);
19) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, 3000 or more, 3200 or more, 3500 or more, or 3700 or more consecutive nucleotides, e.g., 100 to 3057, 200 to 3057, 500 to 3057, 700 to 3057, 900 to 3057, 1000 to 3057, 1200 to 3057, 1400 to 3057, 1500 to 3057, 1700 to 3057, 2000 to 3057, 2200 to 3057, 2500 to 3057, 2700 to 3057, 3000 to 3057, or 3057 consecutive nucleotides within the sequence of SEQ ID NO: 10;
20) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 19);
21) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 100 to 3313, 200 to 3313, 500 to 3313, 700 to 3313, 900 to 3313, 1000 to 3313, 1200 to 3313, 1400 to 3313, 1500 to 3313, 1700 to 3313, 2000 to 3313, 2200 to 3313, 2500 to 3313, 2700 to 3313, 3000 to 3313, or 3313 consecutive nucleotides within the sequence of SEQ ID NO: 11;
22) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 21);
23) a polynucleotide comprising 100 or more, 200 or more, 500 or more, 700 or more, 900 or more, 1000 or more, 1200 or more, 1400 or more, 1500 or more, 1700 or more, 2000 or more, 2200 or more, 2500 or more, 2700 or more or 3000 or more consecutive nucleotides, e.g., 100 to 3375, 200 to 3375, 500 to 3375, 700 to 3375, 900 to 3375, 1000 to 3375, 1200 to 3375, 1400 to 3375, 1500 to 3375, 1700 to 3375, 2000 to 3375, 2200 to 3375, 2500 to 3375, 2700 to 3375, 3000 to 3375, or 3375 consecutive nucleotides within the sequence of SEQ ID NO: 12;
24) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, 2700 or more, or 3000 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, 2700 to 3500, 3000 to 5000, 3000 to 4500, 3000 to 4000, or 3000 to 3500 consecutive nucleotides within Homo sapiens chromosome X inclusive of the polynucleotide 23);
25) a polynucleotide comprising 100 or more, 200 or more, 500 or more, 700 or more, 1000 or more, 1200 or more, 1500 or more, 1700 or more, 2000 or more, 2200 or more, 2500 or more, or 2700 or more consecutive nucleotides, e.g., 100 to 3000, 200 to 3000, 500 to 3000, 700 to 3000, 900 to 3000, 1000 to 3000, 1200 to 3000, 1400 to 3000, 1500 to 3000, 1700 to 3000, 2000 to 3000, 2200 to 3000, 2500 to 3000, 2700 to 3000, or 3000 consecutive nucleotides within the sequence of SEQ ID NO: 13;
26) a polynucleotide comprising 200 or more, 500 or more, 700 or more, 900 or more, 1000 or more, 1200 or more, 1400 or more, 1500 or more, 1700 or more, 2000 or more, 2200 or more, 2500 or more, or 2700 or more consecutive nucleotides, e.g., 200 to 5000, 200 to 4500, 200 to 4000, 200 to 3500, 500 to 5000, 500 to 4500, 500 to 4000, 500 to 3500, 700 to 5000, 700 to 4500, 700 to 4000, 700 to 3500, 900 to 5000, 900 to 4500, 900 to 4000, 900 to 3500, 1000 to 5000, 1000 to 4500, 1000 to 4000, 1000 to 3500, 1200 to 5000, 1200 to 4500, 1200 to 4000, 1200 to 3500, 1400 to 5000, 1400 to 4500, 1400 to 4000, 1400 to 3500, 1500 to 5000, 1500 to 4500, 1500 to 4000, 1500 to 3500, 1700 to 5000, 1700 to 4500, 1700 to 4000, 1700 to 3500, 2000 to 5000, 2000 to 4500, 2000 to 4000, 2000 to 3500, 2200 to 5000, 2200 to 4500, 2200 to 4000, 2200 to 3500, 2500 to 5000, 2500 to 4500, 2500 to 4000, 2500 to 3500, 2700 to 5000, 2700 to 4500, 2700 to 4000, or 2700 to 3500 consecutive nucleotides within Homo sapiens chromosome X inclusive of the polynucleotide 25);
27) a polynucleotide comprising 100 or more, 200 or more, 500 or more, 700 or more, 900 or more, 913 or more, 1000 or more, 1200 or more, 1400 or more, 1405 or more, 1500 or more, 1700 or more, 2000 or more, 2200 or more, 2500 or more, 2700 or more or 3000 or more consecutive nucleotides, e.g., 100 to 3556, 200 to 3556, 500 to 3556, 700 to 3556, 900 to 3556, 913 to 3556, 1000 to 3556, 1200 to 3556, 1400 to 3556, 1405 to 3556, 1500 to 3556, 1700 to 3556, 2000 to 3556, 2200 to 3556, 2500 to 3556, 2700 to 3556, 913, 1405, or 3556 consecutive nucleotides within the sequence of SEQ ID NO: 14;
28) a polynucleotide comprising 200 or more, 500 or more, 700 or more, 900 or more, 913 or more, 1000 or more, 1200 or more, 1400 or more, 1405 or more, 1500 or more, 1700 or more, 2000 or more, 2200 or more, 2500 or more, 2700 or more, 3000 or more, or 3500 or more consecutive nucleotides, e.g., 200 to 5000, 200 to 4500, 200 to 4000, 500 to 5000, 500 to 4500, 500 to 4000, 700 to 5000, 700 to 4500, 700 to 4000, 900 to 5000, 900 to 4500, 900 to 4000, 913 to 5000, 913 to 4500, 913 to 4000, 1000 to 5000, 1000 to 4500, 1000 to 4000, 1200 to 5000, 1200 to 4500, 1200 to 4000, 1400 to 5000, 1400 to 4500, 1400 to 4000, 1405 to 5000, 1405 to 4500, 1405 to 4000, 1500 to 5000, 1500 to 4500, 1500 to 4000, 1700 to 5000, 1700 to 4500, 1700 to 4000, 2000 to 5000, 2000 to 4500, 2000 to 4000, 2200 to 5000, 2200 to 4500, 2200 to 4000, 2500 to 5000, 2500 to 4500, 2500 to 4000, 2700 to 5000, 2700 to 4500, 2700 to 4000, 3000 to 5000, 3000 to 4500, 3000 to 4000, 3500 to 5000, 3500 to 4500, or 3500 to 4000 consecutive nucleotides within Homo sapiens chromosome X inclusive of the polynucleotide 27);
29) polynucleotides comprising complementary sequences to polynucleotides 1) to 28), respectively; and
30) polynucleotides comprising nucleotide sequences having an identity of 60% or more, 65% or more, 70% or more, 75% or more, 80% or more, 85% or more, 90% or more, 92% or more, 95% or more, 97% or more, 98% or more, or 99% or more with the polynucleotides 1) to 29), respectively.

Unless otherwise stated, the polynucleotides 1) to 29) are construed to encompass the scope set forth for the polynucleotides 30).

The term "identity", as used herein in conjugation with polypeptide sequences, refers to the degree of sequence relatedness between two nucleotide sequences as determined by the identity of the match between two strings of the sequences. Identity can be readily calculated. While there exists a number of methods to measure identity between two nucleotide sequences, the term "identity" is well known to skilled artisans. Examples of methods commonly employed to determine identity between two sequences comprise, but are not limited to, GCG (Genetics Computer Group, Madison Wis.) program package, BLASTP, BLASTN, and FASTA. The well-known Smith Waterman algorithm may also be used to determine identity. As an illustration, a nucleic acid comprising a nucleotide sequence having at least, for example, 95% "identity" with a reference nucleotide sequence means that the nucleotide sequence of the nucleic acid is identical to the reference sequence except that the nucleotide sequence may include up to five-point mutations per each 100 nucleotides of the reference nucleotide sequence. In other words, to obtain a nucleotide having a nucleotide sequence at least 95% identical to a reference nucleotide sequence, up to 5% of the nucleotides in the reference sequence may be deleted or substituted with another nucleotide, or a number of nucleotides which may be inserted into the reference sequence amounts to up to 5% of the total nucleotides in the reference sequence. These mutations of the reference sequence may occur at the 5' or 3' terminal positions of the reference nucleotide sequence or anywhere (continuous or discontinuous positions) between those terminal positions.

In an embodiment, the polynucleotide may be a fraction of a nucleotide sequence selected from SEQ ID NOS: 1 to 14 or a 5' terminal fraction thereof. The fraction may comprise the nucleotide sequence of SEQ ID NO: 15 or 16.

The polynucleotide described in the foregoing may retain structural and/or functional traits as a chromatin control element (e.g., a function of controlling the degree of compactness (looseness and tightness) of the surrounding chromosome structure to activate the transcription of genes in a corresponding region).

In an embodiment, the polynucleotide may be at least one selected from the group consisting of polynucleotides comprising the nucleotide sequences of SEQ ID NOS: 1 to 16 and polynucleotides comprising nucleotide sequences complementary the nucleotide sequences of SEQ ID NOS: 1 to 16.

As stated above, the polynucleotide may be used to enhance the expression of a gene coding for a polypeptide of interest upon the introduction of the gene into host cells.

Therefore, contemplated according to another aspect is a polynucleotide, selected from the group consisting of the polynucleotides described in the foregoing, for production or production enhancement of a polypeptide, or a composition containing the at least one of the polynucleotides for production or production enhancement of a polypeptide.

Another aspect provides a recombinant vector comprising a human genomic DNA-derived polynucleotide functioning as the chromatin control element. The recombinant vector may further comprise at least one general gene expression regulatory element such as a promoter, a transcription terminator in addition to the polynucleotide. In an embodiment, the recombinant vector may comprise a promoter, a transcription terminator, and the polynucleotide. In this context, the polynucleotide may be located on the 5' terminal side of the promoter, the 3' terminal side of the transcription terminator, or both thereof.

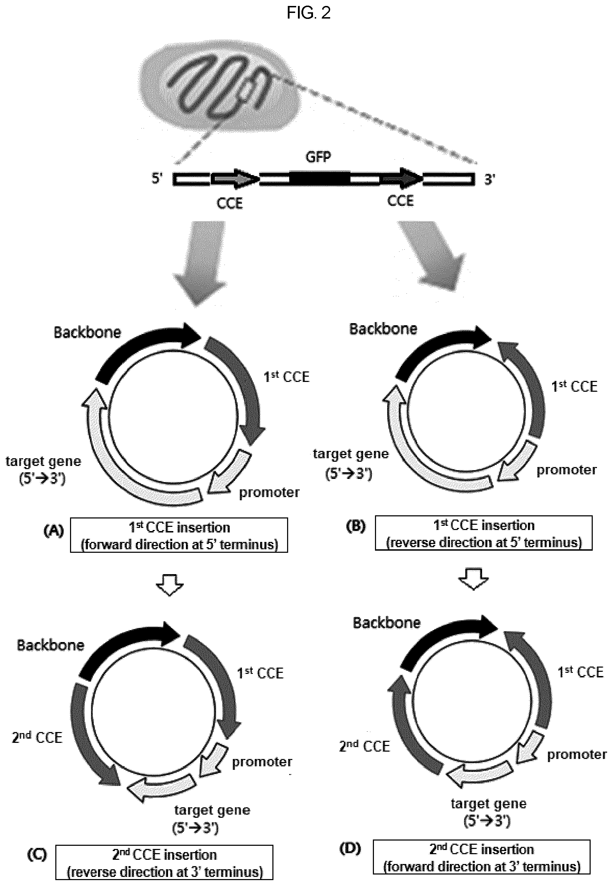

The recombinant vector may comprise one or more (e.g., 1 to 10, 1 to 5, 1 to 3 or 1 to 2) of the polynucleotides on the 5' terminal side of the promoter, the 3' terminal side of the transcription terminator, or both thereof. In this regard, the polynucleotide is as described above. When two or more polynucleotides are employed, they may be independently from the group described above. The two or more polynucleotides may be copies of the same one or may comprise at least one different one to the remainder. In addition, when two or more polynucleotides are used as stated above, they may be independently arranged in the forward (5'→3') or reverse (3' >5') direction. By way of example, when one or more of the polynucleotides are located on the 5' terminal side of the promoter and on the 3' terminal side of the transcription terminator, the polynucleotides on the 5' terminal side of the promoter and on the 3' terminal side of the transcription terminator may be arranged in opposite directions to each other (see FIG. 2), but with no limitations thereto.

In the case where the recombinant vector comprises two or more promoters for two or more inserted genes coding for respective polypeptides of interest, the polynucleotide may be inserted into the 5' terminal side of any one of the two or more promoters and may be one or more (e.g., 1 to 10, 1 to 5, 1 to 3, or 1 to 2) polynucleotidyl entities. When two or more promoters are present, one or more of the polynucleotides may be preferably inserted into the 5' terminal side of a promoter which is located the most upstream of the other promoter(s). In addition, when two or more transcription terminators are employed for two or more genes coding for respective polypeptides of interest in the recombinant vector, the polynucleotide may be inserted into the 3' terminal side of at least one of the two or more transcription terminators and may be one or more (e.g., 1 to 10, 1 to 5, 1 to 3 or 1 to 2) polynucleotidyl entities. When two or more transcription terminators are present, one or more of the polynucleotides may be preferably inserted into the 3' terminal side of a transcription terminator which is located the most downstream of the other transcription terminator(s).

In an embodiment, when two polynucleotides are used, one of the polynucleotides may be inserted in the forward direction into the 5' terminal side of the promoter (i.e., the 5' terminal side of a gene coding for a polypeptide of interest) while the other may be inserted in the reverse direction into the 3' terminal side of a transcription terminator (i.e., the 3' terminal side of a gene coding for a polypeptide of interest). In an alternative embodiment, one polynucleotide may be inserted in the reverse direction into the 5' terminal side of the promoter (i.e., the 5' terminal side of a gene coding for a polypeptide of interest) while the other may be inserted in the forward direction into the 3' terminal side of a transcription terminator (i.e., the 3' terminal side of a gene coding for a polypeptide of interest).

The wording "a polynucleotide is inserted into (and/or contained on and/or located on) the 5' terminal side of a promoter", as used herein in conjugation with the recombinant vector, means that a polynucleotide is connected directly to the 5' terminus of the promoter or located upstream of the promoter. Also, the wording "a polynucleotide is inserted into (and/or contained on and/or located on) the 3' terminal side of a transcription terminator", as used herein in conjugation with the recombinant vector, means that a polynucleotide is connected directly to the 3' terminus of the transcription terminator or located downstream of the transcription terminator.

The recombinant vector may further comprise a gene coding for a polypeptide of interest between a promoter and a transcription terminator. In this regard, the recombinant vector may be used as an expression vector for expressing the gene coding for a polypeptide of interest. The gene coding for a polypeptide of interest may be a foreign gene derived from cells homologous or heterologous to a host cell.

In the recombinant vector, gene expression regulatory elements, such as a promoter, a transcription termination sequence, etc., may be operatively linked to a gene coding for a polypeptide of interest. The term "operatively linked" means a functional linkage (cis) between a gene expression regulatory sequence and a nucleotide sequence to be expressed. A gene expression regulatory sequence can regulate the transcription and/or translation of a nucleotide sequence when the gene expression regulatory sequence is operatively linked to the nucleotide sequence. In order that gene expression regulatory elements are operatively linked to a gene coding for a polypeptide of interest (interchangeably used with a target gene) in the recombinant vector, the promoter among the gene expression regulatory elements may be located at the 5' terminal side of the gene coding for a polypeptide of interest while the transcription terminator may be located at the 3' terminal side of the gene coding for a polypeptide of interest.

In the recombinant vector, the polynucleotide of the present disclosure may be operatively linked to the gene expression regulatory elements/sequences and/or a gene coding for a polypeptide of interest.

The promoter, which is one of transcription regulatory sequences, is a polynucleotide fragment with a size of about 100 to about 2000 bp or about 100 to about 2500 bp, functioning to regulate the transcriptional initiation of a certain gene. In an embodiment, any promoter may be available as long as it can regulate transcriptional initiation in cells, for example, viral cells, bacterial cells, eucaryotes, insect cells, plant cells, or animal cells (e.g., mammalian cells). For example, the promoter may be at least one selected from the group consisting of promoters of prokaryotes or mammalian viruses, such as a cytomegalovirus (CMV) promoter (e.g., CMV immediate-early promoter), a SV40 promoter, an adenovirus promoter (major late promoter), a pL^{λ} promoter, a *trp* promoter, a lac promoter, a tac promoter, T7 promoter, a vaccinia virus 7.5K promoter, a HSV *tk* promoter, a SV40E1 promoter, a respiratory syncytial virus (RSV) promoter, etc., and promoters of animal cells, such as a metallothionin promoter, a β-actin promoter, a γ-actin promoter, a ubiquitin C promoter, an elongation factor 1-α (EF1-α) promoter, a human interleukin-2 (IL-2) promoter, a human lymphotoxin promoter, a glyceraldehyde-3-phosphate dehydrogenase (GAPDH) promoter, a ribosomal protein L32 (RPL32) promoter, a phosphoglycerate kinase (PGK) promoter, a human granulocyte-macrophage colony stimulating factor (GM-CSF) promoter, an albumin promoter (liver-specific), a lymph-specific promoter, a T cell receptor promoter, a neuron-specific promoter (e.g., neurofilament promoter, etc.), a pancreas-specific promoter, a mammalian gland-specific promoter (e.g., milk whey promoter), a mouse hox promoter, and a thea-fetoprotein promoter, but with no limitations thereto.

In one embodiment, the promoter may be a human CMV promoter, a mouse CMV promoter, a human ubiquitin C promoter, or a fusion promoter in which any one of the promoters is fused with an intron. The fusion promoter can remarkably improve the expression efficiency of a gene operatively linked thereto.

The transcription terminator may be a polyadenylation sequence (pA), etc.

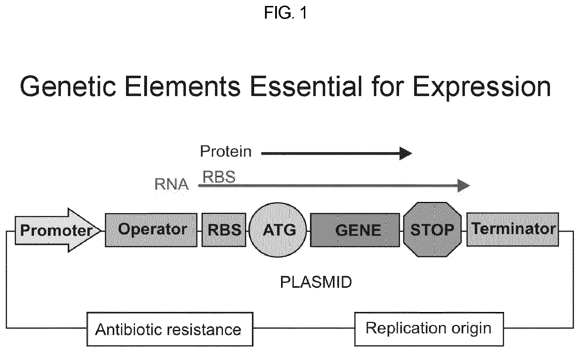

The term "vector" refers to any means for expressing a gene of interest in a host cell. The vector may comprise elements necessary for expressing a gene of interest, such as a replication origin, a promoter, an operator, a transcription terminator, and the like. In addition, a vector may further comprise an enzyme site for introducing a foreign gene into a genome of a host cell (e.g., a restriction enzyme site), a selection marker for confirming a successful introduction of the vector into a host cell, and/or a ribosome binding site (RBS) for translation to a protein (see FIG. 1). The recombinant vector may further comprise a transcription regulatory sequence (e.g., an enhancer) in addition to a promoter.

The origin of replication may be an f1 origin of replication, an SV40 origin of replication, a pMB1 origin of replication, an adeno origin of replication, an AAV origin of replication, or a BBV origin of replication.

Also, the recombinant vector may further comprise a selection marker. The selection marker is a gene for confirming whether or not the recombinant vector is successfully introduced into a host cell or for establishing a stable recombinant cell comprising the recombinant vector. For example, the selection marker may be at least one selected from the group consisting of drug-resistant genes, metabolism-related genes, gene-amplifying genes, and the like. So long as it is usually used as a selection marker, any gene (e.g., antibiotic-resistant genes and/or metabolism-related enzyme genes, etc.) may be available without limitations because the selection marker is not a factor that significantly affects the expression efficiency according to the optimal combination in the vector, which is the core technology of the present disclosure. For example, the selection marker may be at least one selected from the group consisting of an ampicillin-resistant gene, a tetracycline-resistant gene, a kanamycin-resistant gene, a chloramphenicol-resistant gene, a streptomycin-resistant gene, a neomycin-resistant gene, a blasticidin-resistant gene, a zeocin-resistant gene, a hygromycin-resistant gene, a puromycin-resistant gene, a thymidine kinase (TK) gene, a dihydrofolate reductase (DHFR) gene, a glutamine synthetase (GS) gene, and the like, but not be limited thereto.

The vector may be exemplified by a plasmid vector, a cosmid vector, or a viral vector such as a bacteriophage vector, an adenovirus vector, a retrovirus vector, and an adeno-related virus vector. The recombinant vector may be constructed from, but not limited to, well-known plasmids (for example, pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, pUC19, etc.), phages (for example, λgt4λB, λ-Charon, λΔz1, M13, etc.) or viruses (for example, SV40, etc.) by manipulation.

Another aspect provides a recombinant cell anchoring the recombinant vector thereat.

The recombinant cell may refer to a cell established by introducing the recombinant vector into a host cell. In this regard, the recombinant vector may carry a gene coding for a polypeptide of interest.

So long as it enables the promoter to operate in the recombinant vector (i.e., function to initiate transcription) and allows for the integration (insertion) of the polynucleotide into the chromosome and/or the expression of a gene coding for a polypeptide of interest, any eukaryotic (e.g., mammalian) cell may be used as the host cell. By way of example, the mammalian host cell suitable for use in the present disclosure may be at least one selected from the group consisting of a murine cell (e.g., COP, L, C127, Sp2/0, NS-0, NS-1, At20, or NIH3T3), a rat cell (e.g., PC12, PC12h, GH3, or MtT), a hamster cell (e.g., BHK, CHO, GS gene-deficient CHO, or DHFR gene-deficient CHO), a monkey cell (e.g., COS1, COS3, COST, CV1, or Vero), a human cell (e.g., Hela, HEK-293, PER C6 cell derived from retinal tissue, a cell derived from diploid fibroblast, myeloma cell, or HepG2), and the like, but not limited thereto.

The delivery (introduction) of a recombinant vector into a host cell may be easily carried out by any method known in the relevant art. The genetic introduction may be performed using, but not limited to, microinjection, calcium phosphate precipitation, electroporation, liposome-mediated transfection, or particle bombardment.

A recombinant cell (transformant) having the expression vector introduced thereinto may be selected by any well-known method using a typical selection marker. For example, when the selection marker is a gene resistant to a certain antibiotic as described above, the cells may be grown in the presence of the antibiotic in a medium to select a recombinant cell into which the recombinant vector has successfully been introduced.

When a gene coding for a polypeptide of interest is expressed in a host cell transformed with a recombinant vector, the presence of the polynucleotide of the present disclosure in the recombinant vector can increase the expression level by 20% or more (1.2 times or more), 30% or more (1.3times or more), 40% or more (1.4 times or more), 50% or more (1.5 times or more), 60% or more (1.6 times or more), 70% or more (1.7 times or more), 80% or more (1.8 times or more), 90% or more (1.9 times or more), 100% or more (2 times or more), 110% or more (2.1 times or more), 120% or more (2.2 times or more), 130% or more (2.3 times or more), 140% or more (2.4 times or more), 150% or more (2.5 times or more), or 200% or more (3 times or more), compared to the absence of the polynucleotide.

Another aspect provides a use of the recombinant vector carrying the polynucleotide and/or the recombinant cell anchoring the recombinant vector thereat for production and/or production enhancement of a polypeptide of interest. In detail, provided is a composition for production or production enhancement of a polypeptide of interest, the composition comprising; a recombinant vector carrying the polynucleotide, transcription regulatory sequences (promoter, transcription terminator, and the like), and a gene, operatively linked thereto, coding for a polypeptide of interest; a recombinant cell anchoring the recombinant vector; or a combination thereof.

Another aspect provides a method for production or production enhancement of a polypeptide of interest, using the recombinant vector or the recombinant cell.

In an embodiment, the production method or production enhancement method may comprise a step of expressing a gene coding for a polypeptide of interest in the recombinant cell. The step of expressing a gene may be carried out in vitro. The step of expressing a gene may comprise a step of culturing the recombinant cells in a cell culture medium under a condition permissive for the expression of the gene.

The production or production enhancement method may further comprise a step of introducing the recombinant vector into a host cell prior to the expression step or the culturing step. The recombinant vector comprises the polynucleotide of the present disclosure and transcription regulatory sequences and may further comprise a gene coding for a polypeptide of interest.

In addition, the production or production enhancement method may further comprise a step of harvesting (obtaining or separating) the polypeptide of interest from the expression or culture product, after the expressing or culturing step. The method may further comprise a purification step for removing impurities, for example, nucleic acids, viruses, host cell proteins, etc., from the culture. The purification step may be performed using any typical method, for example, affinity chromatography (protein A affinity chromatography, protein B affinity chromatography, KappaSelect affinity chromatography C, etc.), ion exchange chromatography (anion exchange chromatography, cation exchange chromatography, etc.), hydrophobic interaction or aromatic adsorption chromatography, size exclusion chromatography, electrophoresis, and the like, but with no limitations thereto.

Another aspect provides a method for production or production enhancement of a polypeptide of interest in a host cell or a method for preparation of a recombinant cell for producing a polypeptide of interest, the method comprising a step of introducing the above-described recombinant vector.

As used herein, the term "polypeptide" refers to a molecule covering a polymer of amino acids which are linked to one another through peptide bond(s). The polypeptide may a polypeptide in any length; for example, the polypeptide may comprise two or more amino acid residues, i.e., may be a protein or an oligopeptide.

As used herein, the term "polypeptide of interest" in the expression "gene coding for a polypeptide of interest" refers to a protein or a peptide having a desired activity in vivo (e.g., the activity of preventing, alleviating, and/or treating a certain disease or symptom, and/or replacing a substance necessary in a living body). For example, the polypeptide of interest may be at least one selected from the group consisting of a protein or peptide having an enzymatic activity (e.g., a protease, a kinase, a phosphatase, etc.), a receptor protein or peptide, a transporter protein or peptide, a microbiocidal and/or endotoxin-binding polypeptide, a structural protein or peptide, an immune polypeptide (e.g., immunoglobulin), a toxin, an antibiotic, a hormone, a growth factor, a vaccine, and the like. The polypeptide of interest may be endogenous or exogenous (from identical or different species). The polypeptide of interest may be intrinsic or extrinsic, and in the case where the polypeptide is extrinsic, it may be derived from cells homologous or heterogenous to the host cell.

In an embodiment, the polypeptide of interest may be at least one selected from the group consisting of a hormone, a cytokine, a tissue plasminogen activator, an immunoglobulin (e.g., an antibody or an antigen-binding fragment thereof or a variant thereof), and the like. The immunoglobulin (antibody) may be any isotype (e.g., IgA, IgD, IgG, IgM, or IgE), for example, an IgG (e.g., IgG1, IgG2, IgG3, or IgG4) molecule. In an embodiment, the antibody may be construed to encompass antibody analogs in fusion protein forms where an antibody constant region (e.g., Fc, CH2-CH3 domains, etc.) is combined with the entirety or part of a ligand or receptor. The antigen-binding fragment refers to an antibody fragment possessing an antigen binding ability of the antibody, and may comprise or consist essentially of at least about 20 amino acids, for example, at least about 100 amino acids. The antigen-binding fragment may be any fragment containing an antigen-binding region, and for example, it may be at least one selected from the group consisting of CDRs, Fab, F(ab)2, Fv, scFv, a multibody containing various antigen-binding domains (e.g., a diabody, a triabody, a tetrabody, etc.), a single-domain antibody, an affibody, and the like. The variant of an antibody refers to a derivative of an antibody or an antibody fragment, which has an amino acid sequence modified from the amino acid sequence of an original antibody, but retains the same antigen-binding affinity as in the original antibody. The antibody and/or antigen-binding fragment may be, but not limited to, animal antibodies, chimeric antibodies, humanized antibodies, or human antibodies. In addition, the antibody may be a mono-specific antibody, a bi-specific antibody, or a multi-specific antibody. For instance, the antibody may be an antibody or a fragment thereof that can bind to any one antigen selected from among HER2, HER3, CD33, VEGF, VEGFR, VEGFR-2, EGFR, CD152, CD40, TNF, IL-1, IL-5, IL-17, IL-6R, IL-1, IL-2R, BLYS, OX40L, CTLA4, PCSK9, EGFR, c-Met, CD2, CD3, CD11a, CD19, CD30, CD38, CD20, CD52, CD60, CD80, CD86, TNF-α, IL-12, IL-17, IL-17A, IL-23, IL-6, IL-6R, IL-1β, IL-4R α, RSVF, IgE, RANK, BLyS, α4β7, PD-1, PD-L1, CCR4, SLAMF7, GD2, CD21, CD79b, IL20Rα, CD22, CD79a, CD72, IGF-1R, RANKL, PD-1, PD-L1, factor IXa, factor X, and cMET. More specifically, the antibody may be at least one selected from the group consisting of adalimumab, aflibercept, abciximab, omalizumab, palivizumab, basiliximab, eculizumab, certolizumab, infliximab, golimumab, etanercept, abatacept, tocilizumab, denosumab, ustekinumab, bevacizumab, ranibizumab, alemtuzumab, rituximab, cetuximab, panitumumab, trastuzumab, pertuzumab natalizumab, nivolumab, atezolizumab, daratumumab, secukinumab, pembrolizumab, dupilumab, emicizumab, durvalumab, guselkumab, and tocilizumab, but with no limitations thereto.

The antibody or antigen-binding fragment may be isolated from a living body or non-naturally occurring (e.g., recombinantly or synthetically produced). When the polypeptide of interest is an antibody or antigen-binding fragment, a gene encoding a heavy chain (heavy chain CDR, heavy chain variable region, and/or heavy chain encoding gene) and a gene encoding a light chain (light chain CDR, light chain variable region, and/or light chain encoding gene) may be carried together in one vector, or separately in different vectors into a host cell.

In light of the definition of the polypeptide of interest as described above, a person skilled in the art can obviously know the specific details (e.g., base sequence, etc.) of the gene coding for the polypeptide of interest.

### Advantageous Effects

By providing a chromatin control element for high expression of therapeutic proteins, such as antibodies, in animal cells, the present disclosure can find advantageous applications in the mass production of various therapeutic proteins.

### Description of Drawings

FIG. 1 is a schematic diagram of a recombinant vector for expressing a target gene.
FIG. 2 is a schematic view showing cleavage maps of recombinant vectors for assaying CCE efficacy and cloning processes therefor (in FIG. 2, the target gene accounts for a gene coding for a polypeptide of interest, the first CCE for a CCE inserted into the 5' terminal side of the target gene, and the second CCE for a CCE inserted into the 3' terminal side of the target gene).
FIG. 3 is a graph showing relative antibody expression levels (%) in recombinant cells anchoring CCE sequences to the control (100%).
FIG. 4 is a schematic diagram of core CCE#7 and core CCE#8 according to an embodiment.
FIG. 5a is a plot of absolute values of antibody titers after introduction of expression vectors (w/o CCE, CCE14, core CCE #7, and core CCE #8).
FIG. 5b is a plot of absolute values of Qp after introduction of expression vectors (w/o CCE, CCE14, core CCE #7, and core CCE #8).
FIG. 6a is a plot of antibody titers after introduction of expression vectors (w/o CCE, CCE14, core CCE #7, and core CCE #8) relative to the reference expression vector (CCE14).
FIG. 6b is a plot of Qp after introduction of expression vectors (w/o CCE, CCE14, core CCE #7, and core CCE #8) relative to the reference expression vector (CCE14).
FIGS. 7a and 7b are plots of antibody titer equivalent values of core CCE #7 (FIG. 7a; p value = 0.001) and core CCE #8 (FIG. 7b; p value = 0.000) relative to CCE14, as measured by an equivalence test.

### Mode for Invention

Hereafter, the present invention will be described in detail in the following Examples.

The following examples are intended merely to illustrate the invention and are not construed to restrict the invention.

### EXAMPLE 1: Discovery of Chromatin Control Element (CCE) in Human Genomic DNA

In reporter gene (GFP gene)-overexpressing human cells (Host: 293F, Invitrogen, R790-07), the insertion position of the reporter gene was detected, and a search was made for sequences that could affect the overexpression in the vicinity of the reporter gene. Sequences located around the 5' and 3' termini of the reporter gene were examined.

In brief, GFP-overexpressing clones per batch were selected as follows.

### [Production of retrovirus using GP2-293 cell line]

VC003-1 DNA (pRetroQ-AcGFP1-C1 vector; Clontech, #632506) and VSV-G DNA (VSV-G vector; Clontech, #631530) were transfected into the GP2-293 cell line (Clontech, #631458) with the aid of Lipofectamine^{®} 2000. Following transfection, the cells were cultured at 37°C for 28 hours in a 5% CO₂ atmosphere with 80% humidity. The cell culture thus obtained was allowed to pass through a 0.45-µm cellulose filter to filter the viruses, and the filtrate was stored at 4°C and used within 3 days. The filtered culture was used within 3 days while being stored at 4°C.

### [Viral transduction]

293F cells (Invitrogen, R790-07) were treated with the virus filtrate obtained above at 37°C for 24 hours in a 5% CO₂ atmosphere with 80% humidity. Then, the medium was replaced by DMEM + 10% FBS in which the cells were cultured.

### [Acquisition of monoclone]

The transduced 293F cells were subjected to geneticin selection, followed by FACS sorting to select a pool of high-expression cells.

The selected pool of high-expression cells was maintained in DMEM/F12 medium (Life technology, A1 1320-033) supplemented with 10% FBS (Life technology, 16000-044) and geneticin (Life technology, 10131-027). In order to obtain monoclones from the pool, the cells were counted with a cell counter (Roche Innovatis AG, Cedex standard plus M20-C), and then, limiting dilution was performed. As a result, a total of 120 monoclones were acquired.

### [Examination of high expression level of GFP]

With respect to the acquired 120 clones, living cells were stained with DAPI (Invitrogen, Hoechst 33348) and screened by an Incell analyzer (GE Healthcare, Incell analyzer 6000). As a result, the top 24 GFP-expressing clones were selected in the order of the highest GFP expression level per unit cell.

### [Confirmation of high expression level of GFP]

For the selected 24 clones, the top 6 clones per batch were selected by measuring the GFP expression level again using FACS (BD, FACS canto II) according to the method provided by BD.

### [Examination of single copy insertion clone]

In order to examine whether the high expression of GFP of the selected top 6 clones is due to single copy insertion, a genome copy number assay was performed using real-time PCR. Selection was made of clones with a genome copy number of less than 1.05 as measured by real-time PCR.

### [Search for flanking region]

Tail-PCR (Thermal Asymmetric Interlaced PCR) was performed using the KOD-Plus-Neo PCR kit (Cat# KOD-401, TOYOBO, Japan) to find the flanking region around the GFP in the selected clones. A band portion comprising the flanking region was purified from the agarose gel, using a gel extraction kit (QIAGEN, Cat. 28706). The purified amplicon thus obtained was cloned into pJet, using the pJet1.2 kit (Cat# K1231, Thermo Scientific, USA). The obtained sequence was analyzed by NCBI BLAST to obtain a gDNA sequence about 100 kb long. The gDNA sequence was analyzed through the MAR finder program. As a result, 14 chromatin control element (CCE) sequences were discovered (SEQ ID NOS: 1 to 14; hereinafter referred to as CCE1 to CCE14).

### EXAMPLE 2: Construction of Recombinant Vector Carrying Human Chromatin Control Element (CCE) and Antigen Gene and Assay for Antibody Expression Level

The 14 CCE sequences obtained in Example 1 were each inserted in the forward direction into the 3' terminal side of the antigen gene and/or in the reverse direction into the 5' terminal side of the antigen gene within the vector to construct expression vectors comprising the human genome-derived chromatin control element (CCE) sequences, and the vectors were assayed for expression performance. When two CCE sequences were inserted into one vector, the CCE inserted into the 5' terminal side of the antibody gene was named first CCE while the CCE inserted into the 3' terminal side of the antibody gene was named second CCE.

The 14 CCE sequences obtained in Example 1 were discovered on the 5' or 3' terminal side of the position where the reporter gene (GFP) was inserted.

### [Construction of vector for expressing polypeptide of interest]

The omalizumab antibody (Accession number DB00043) was used as a target polypeptide. The omalizumab antibody was approved by the FDA in June 2003 and has been commercially available under the trademark Xolair^{™} since then, and its antibody sequence is already disclosed in US6329509 or DrugBank (https://www.drugbank.ca/drugs/DB00043).

First, a promoter and an omalizumab antibody gene were treated with Mlul (NEB, R0198L), Pvul (NEB, R0150L), Ascl (NEB, R0558L), and Pvul (NEB, R0150L) and then cloned into pcDNA^{™}3.3 vector (Life technology) to construct a vector for expressing the target polypeptide.

### [Acquisition of CCE insert and Construction of vector carrying CCE]

After synthesis of primers (SEQ ID NOS: 17 to 48) for nucleotide sequences of a certain region (±2kb) around the CCEs obtained in Example 1, vectors carrying the CCEs and omalizumab were constructed using the polymerase KOD FX Neo (TOYOBO Bio-Technology, Cat.No KFX-201) according to the protocol provided by the manufacturer, with the CHO-K1 gDNA serving as a template. The CCE inserts were ligated to the vectors, using the ligation kit (Roche, Rapid ligation kit, cat.No.11635379001). For ligation, the vector and the insert were digested with Mull. In this regard, the CCE insert that existed on the 5' terminal side of the position at which the reporter gene (GFP) was inserted upon the discovery of CCE on the genomic DNA was inserted in the forward direction into the 5' terminal side of the antibody gene and in the reverse direction into the 3' terminal side of the antibody gene (see (C) in FIG. 2). The CCE insert that existed on the 3' terminal side of the position at which GFP was inserted in the forward direction into the 3' terminal side of the antibody gene and in the reverse direction into the 5' terminal side of the antibody gene (see (D) in FIG. 2). For example, the CCE insert (CCE14) was inserted in the forward direction into the 3' terminal side of the omalizumab antibody and in the reverse direction into the 5' terminal side of the omalizumab antibody.

A vector comprising each of CCE1 to CCE13 obtained in Example 1 was constructed in the same manner as the method for preparing a vector comprising CCE14 described above.

### [Comparison of antibody expression level among CCEs]

The human-derived CCE sequence-inserted expression vectors obtained above were introduced into the genome of CHO-K1 cells (ATCC, CRL-9618), with the aid of Lipofectamine^{®}2000 transfection reagent (Cat#11668019, Life Technologies, USA). Following transfection with the CCE vectors, puromycin selection was performed on CHO-K1 cells. The cells were counted using the CEDEX STD cell counter (ROCHE INNOVATIS, USA) and then cultured at 37°C for 72 hours. The resulting culture medium was subjected to an antibody titer experiment using the ForteBio Octet^{®} QK384. Antibody concentrations were measured using a protein A sensor and the measurements were analyzed with the Octet^{®} analysis program.

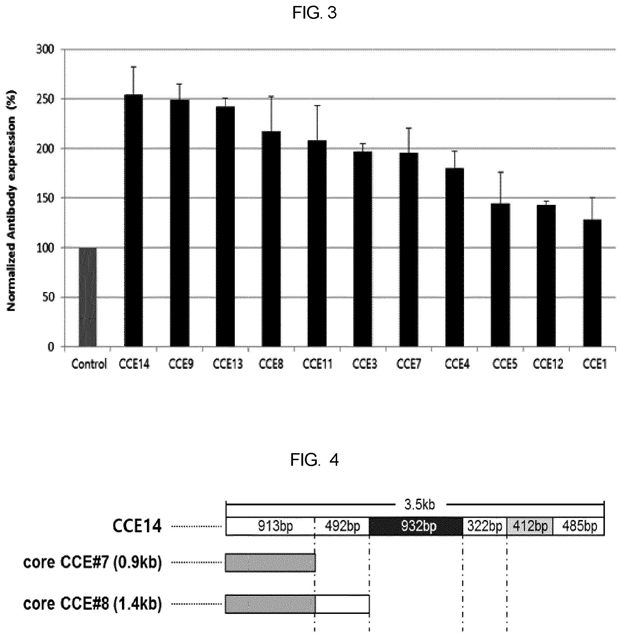

Antibody expression levels (%) in the recombinant cells comprising respective CCE sequences were compared to that in the control (100%, cells having no CCE sequences) and the results are given in Table 1 and depicted in FIG. 3.

**TABLE 1**

| ID | Fold | SD |
|---|---|---|
| Control | 100 | |
| **CCE1** | 128 | 22 |
| **CCE3** | 197 | 8 |
| **CCE4** | 180 | 17 |
| **CCE5** | 144 | 32 |
| **CCE7** | 195 | 25 |
| **CCE8** | 217 | 35 |
| **CCE9** | 249 | 16 |
| **CCE11** | 208 | 35 |
| **CCE12** | 143 | 4 |
| **CCE13** | 242 | 9 |
| **CCE14** | 254 | 28 |

As can be seen in Table 1 and FIG. 3, the CCE sequences obtained in Example 1 showed significantly higher antibody expression rates than in the control (increased by at least 20%, compared to the control).

### EXAMPLE 3: Construction of Recombinant Vector Carrying Partial Human Chromatin Control Element (CCE) and Assay for Antibody Gene Expression Level

From among the human CCEs that were confirmed to have expression effects omalizumab antibody in Example 2, CCE14 (SEQ ID NO: 14) was selected, and its partial fragments (named core CCE14 #7 and core CCE14 #8, respectively) were tested. Core CCE14 #7 (SEQ ID NO: 15), which is a partial fragment of CCE14, is 913-bp long starting from the 5' terminus of CCE14 (SEQ ID NO: 14, full length 3.5kb) (polynucleotide extending from the 1^{st} to the 913^{th} nucleotide on the sequence of SEQ ID NO: 14; 0.9 kb hCCE). Core CCE14 #8 (SEQ ID NO: 16) is 1405-bp long starting from the 5' terminus of CCE14 (SEQ ID NO: 14) (polynucleotide extending from the 1^{st} to 1450^{th} nucleotides on the sequence of SEQ ID NO: 14; 1.4 kb hCCE) (see FIG. 4).

The omalizumab antibody was used as a target polypeptide as in Example 2. A vector for expressing the target polypeptide was constructed and transfected into CHO cells in the same manner as in Example 2, followed by assaying the cells for expression levels of omalizumab.

Briefly, for use as a vector for expressing the target polypeptide, a CCE14 vector was designed to include a hamster EF-1α promoter and CCE14 (SEQ ID NO: 14) in the reverse direction on the 5' terminal side of the antibody gene (first CCE) and in the forward direction on the 3' terminal side of the antibody gene (second CCE) (see (D) in FIG. 2). Likewise, an expression vector was constructed to include the core CCE14 #7 (SEQ ID NO: 15) in the reverse direction on the 5' terminal side of the gene coding for the polypeptide of interest and in the forward direction on the 3' terminal side of the gene coding for the polypeptide of interest (hereinafter, referred to as "core CCE14 #7 vector" or "core CCE #7 vector"). In addition, an expression vector was constructed to include core CCE14 #8 (SEQ ID NO: 16) in the reverse direction on the 5' terminal side of the gene coding for the polypeptide of interest and in the forward direction on the 3' terminal side of the gene coding for the polypeptide of interest (hereinafter, referred to as "core CCE14 #8 vector" or "core CCE #8 vector").

The vectors constructed above were transinfected by electroporation (Amaxa^{™} 4D-Nucleofector^{™} Protocol, SF kit) into CHO cells. On day 4 after transfection, the cells were measured for viable cell density (VCD) and cell viability using the CEDEX STD cell counter (ROCHE INNOVATIS, USA). Also, the cells were assayed for antibody titer and absolute and relative values of productivity (Qp) using ForteBio Octet^{®} QK384, and the measurements were compared and analyzed.

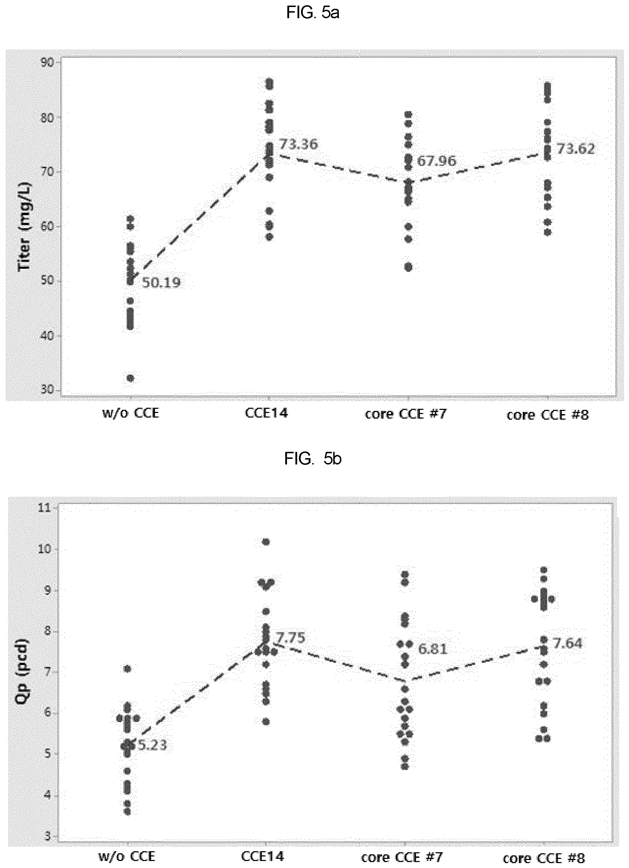

The data thus obtained are summarized in Tables 2 and 3 and depicted in FIGS. 5a to 7b (n=5):

**TABLE 2**

| Analysis of Antibody Titer Ratio (result obtained upon introduction of CCE14 set to be 1) | | | | |
|---|---|---|---|---|
| | w/o CCE | CCE14 core | CCE14 | #7core CCE14 #8 |
| Average of 1^{st} antibody titer ratio | 0.68 | 1.00 | 1.05 | 1.18 |
| SD | 0.12 | 0.09 | 0.04 | 0.03 |
| Average of 2^{nd} antibody titer ratio | 0.76 | 1.00 | 0.92 | 1.03 |
| SD | 0.04 | 0.08 | 0.04 | 0.07 |
| Average of 3^{rd} antibody titer ratio | 0.67 | 1.00 | 0.95 | 0.93 |
| SD | 0.03 | 0.03 | 0.06 | 0.14 |
| Average of 4^{th} antibody titer ratio | 0.62 | 1.00 | 0.78 | 0.89 |
| SD | 0.04 | 0.12 | 0.07 | 0.04 |
| **Total Average** | **0.68** | **1.00** | **0.93** | **1.00** |
| Total SD | 0.10 | 0.11 | 0.11 | 0.11 |

**TABLE 3**

| Analysis of Qp (Productivity) Ratio (result obtained upon introduction of CCE14 set to be 1) | | | | |
|---|---|---|---|---|
| | w/o CCE | CCE14 | core CCE14 #7 | core CCE14 #8 |
| Average of 1^{st} Qp ratio | 0.68 | 1.00 | 0.97 | 1.00 |
| SD | 0.06 | 0.09 | 0.06 | 0.04 |
| Average of 2^{nd} Qp ratio | 0.77 | 1.00 | 0.90 | 1.07 |
| SD | 0.09 | 0.07 | 0.03 | 0.06 |
| Average of 3^{rd} Qp ratio | 0.71 | 1.00 | 0.94 | 1.11 |
| SD | 0.06 | 0.04 | 0.06 | 0.07 |
| Average of 4^{th} Qp ratio | 0.54 | 1.00 | 0.69 | 0.77 |
| SD | 0.05 | 0.16 | 0.05 | 0.05 |
| **Total Average** | **0.68** | **1.00** | **0.88** | **0.99** |
| Total SD | 0.12 | 0.15 | 0.18 | 0.18 |

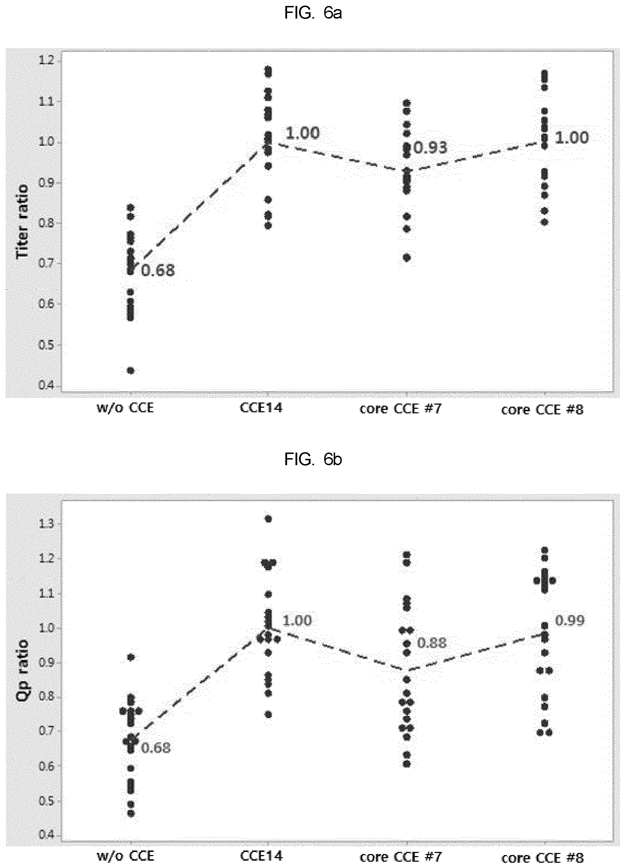

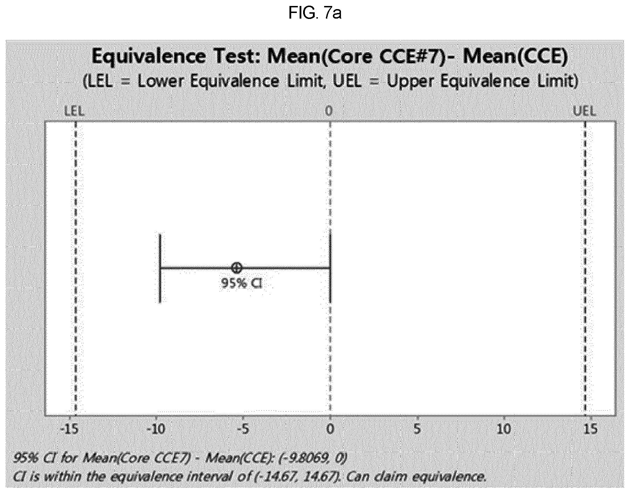

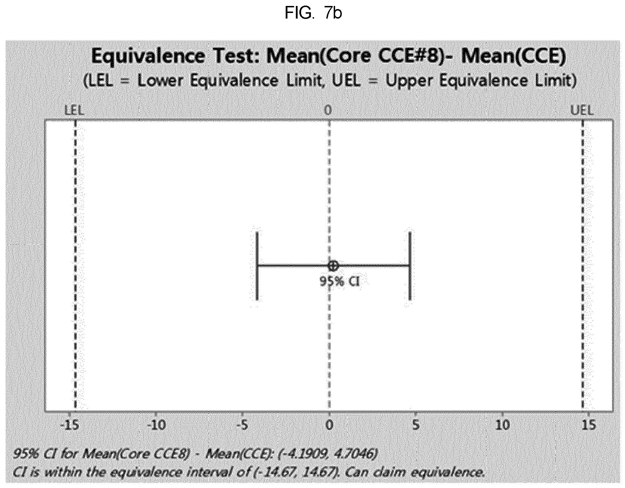

FIGS. 5a and 5b show absolute values of antibody titers and Qp measured after introduction of the expression vectors, respectively. FIGS. 6a and 6b show relative values of antibody titers and Qp measured after introduction of the expression vectors (relative to the values measured after introduction of CCE14 vector), respectively. FIGS. 7a and 7b show titers of the core CCE14 #7 vector and the core CCE14 #8 vector relative to that of CCE14 vector, as measured by an equivalence test.

As can be understood from the data, the CCE14 #7 vector including the 0.9-kb CCE fragment (core CCE14 #7) and the core CCE14 #8 vector including the 1.4-kb CCE fragment (core CCE14 #8) both exhibited similar average titer values (absolute and ratio) and Qp values (absolute and ratio) to those of the CCE14 vector including the full-length CCE14 (3.5 kb). In addition, the core CCE14 #7 vector and the core CCE14 #8 vector were both found to be equivalent in antibody titer to the CCE14 vector as measured by an equivalent test. These results indicate that the CCE fragments as well as the full-length CCE can fulfill the functional role of CCEs.

## Claims

1. A recombinant vector, comprising at least one polynucleotide selected from the group consisting of:
a) a polynucleotide comprising 100 or more consecutive nucleotides within a nucleotide sequence selected from SEQ ID NOS: 1 to 16;
b) a polynucleotide comprising 200 to 5,000 consecutive nucleotides, inclusive of the polypeptide a), on human chromosome X; and
c) a polynucleotide comprising a nucleotide sequence complementary to the polynucleotide a) or b)

2. The recombinant vector of claim 1, wherein the polynucleotide comprises a nucleotide sequence selected from SEQ ID NOS: 1 to 16, a nucleotide sequence complementary thereto, or both of the nucleotide sequence and the complementary sequence.

3. The recombinant vector of claim 1, further comprising a promoter and a transcription terminator.

4. The recombinant vector of claim 3, wherein the polynucleotide is located at a 5' terminal side of the promoter, at a 3' terminal side of the transcription terminator, or at both of the terminal sides.

5. The recombinant vector of claim 4, wherein the polynucleotide is located in opposite directions at both of the terminal sides.

6. The recombinant vector of claim 4, comprising one to five of the polynucleotides on the 5' terminal side of the promoter, on the 3' terminal side of the transcription terminator, or on both of the terminal sides, wherein the one to five of the polynucleotides are the same or at least one of them is different from the remainder.

7. The recombinant vector of any one of claims 1 to 6, further comprising a gene coding for a polypeptide of interest.

8. A recombinant cell, prepared by introducing the recombinant vector of any one of claims 1 to 6 into a host cell.

9. The recombinant cell of claim 8, wherein the recombinant vector further comprises a gene coding for a polypeptide of interest.

10. The recombinant cell of claim 8, wherein the host cell is a eukaryotic animal cell.

11. A method for producing a polypeptide of interest, the method comprising the steps of:
expressing a gene coding for the polypeptide of interest in the recombinant cell of claim 9; and
recovering the expressed polypeptide of interest.

12. A method for producing a polypeptide of interest in a host cell, the method comprising a step of introducing the recombinant vector of claim 7 into the host cell.
